# EUROPÄISCHE PATENTSCHRIFT

(11) **EP 0 863 880 B1**
(45) Veröffentlichungstag und Bekanntmachung des Hinweises auf die Patenterteilung: **19.11.2003**
(21) Anmeldenummer: 96927607.0
(22) Anmeldetag: 26.07.1996
(51) Int. Cl.: C07D 233/88, A61K 31/415

(54) **NEUE, ANTIKONVULSIV WIRKENDE 1-AR(ALK)YL-IMIDAZOLIN-2-ONE, DIE IN 4-STELLUNG EINEN DISUBSTITUIERTEN AMIN-REST ENTHALTEN, UND VERFAHREN ZU DEREN HERSTELLUNG**
NOVEL 1-AR(ALK)YL-IMIDAZOLIN-2-ONES CONTAINING A DISUBSTITUTED AMINE RADICAL IN THE 4TH POSITION, HAVING AN ANTI-CONVULSIVE EFFECT, AND PROCESS FOR THEIR PRODUCTION
NOUVELLES 1-AR(ALK)YL-IMIDAZOLIN-2-ONES A EFFET ANTICONVULSIF CONTENANT UN RESTE AMINE BISUBSTITUE EN POSITION 4 ET PROCEDE PERMETTANT DE LES PREPARER

(30) Priorität: 05.09.1995 DE 19532668
(43) Veröffentlichungstag der Anmeldung: 16.09.1998
(73) Patentinhaber: elbion AG, 01445 Radebeul (DE)
(72) Erfinder: LANKAU, Hans-Joachim, D-01689 Weinböhla (DE); MENZER, Manfred, D-01279 Dresden (DE); UNVERFERTH, Klaus, D-01309 Dresden (DE); GEWALD, Karl, D-01217 Dresden (DE); SCHÄFER, Harry, D-01189 Dresden (DE)
(74) Vertreter: Böhm, Brigitte, Dipl.-Chem. Dr.
(86) Internationale Anmeldenummer: EP9603295
(87) Internationale Veröffentlichungsnummer: WO97009314

(56) Entgegenhaltungen:
- US-A- 3 932 452
- US-A- 4 044 021

## Beschreibung

Die Erfindung betrifft 1-Ar(alk)yl-imidazolin-2-one, die in 4-Stellung einen disubstituierten Amin-Rest enthalten, Verfahren zu ihrer Herstellung und ihre Verwendung als pharmazeutische Mittel zur Behandlung von Erkrankungen des Zentralnervensystems, insbesondere von Epilepsien verschiedener Formen.

1-Ar(alk)yl-imidazolin-2-one mit einem unsubstituierten Amin- oder Methylaminrest in 4-Stellung werden nach dem Stand der Technik durch Umsetzung von Ar(alk)ylaminoacetamiden mit Bromcyan hergestellt. Durch N-Alkylierung der so hergestellten 4-Amino-1-ar(alk)yl-imidazolin-2-one werden 3-Alkyl- oder 1-lminoalkyl-3-alkyl-1-ar(alk)yl-imidazolin-2-one erhalten, wobei die Aminogruppe in 4-Position zu einer Iminogruppe tautomerisiert. Eine weitere N-Alkylierung zu Verbindungen der allgemeinen Formel 1 ist deshalb nicht möglich, so daß erfindungsgemäße Verbindungen nach diesem Verfahren nicht hergestellt werden können [USP 4044021; DE 2251354].

1-Ar(alk)yl-imidazolin-2-one mit einem disubstituierten Amin-Rest in 4-Stellung wurden bisher nicht beschrieben.

Bekannt sind eine Vielzahl antikonvulsiv wirksamer Verbindungen. Doch auch heute können noch nicht alle epileptischen Erkrankungen zufriedenstellend behandelt werden.

Der Erfindung liegt also die Aufgabe zugrunde, neue Verbindungen mit günstigen pharmakologischen Eigenschaften zur Verfügung zu stellen, die beispielsweise als antiepileptisch wirkende Arzneimittel einsetzbar sind.

Entsprechend der vorliegenden Erfindung sind diese neuen Verbindungen 1-Ar(alk)yl-imidazolin-2-one der allgemeinen Formel 1 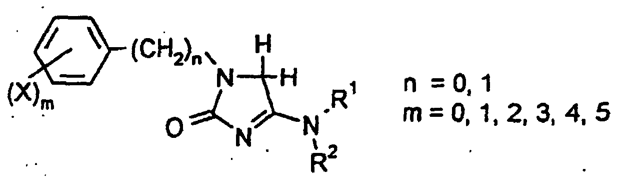 worin
X = Wasserstoff, C₁-C₄-Alkyl, C₁-C₄-Alkoxy, Trifluormethyl, Halogen,
R¹ bzw. R² = C₁-C₄-Alkyl, Cycloalkyl, eine Alkylgruppe, welche ein oder mehrere Heteroatome einschließt, oder
R¹ und R² zusammen eine Alkylengruppe mit 2 - 6 Kohlenstoffatomen, in dem eine -CH₂-Gruppe durch Sauerstoff, Stickstoff oder Schwefel ersetzt sein kann, oder eine 4-Methylpiperazinogruppe bedeuten.

Die Anzahl der CH₂-Gruppen beträgt entweder 0 (1-Aryl-imidazolin-2-one) oder 1 (1-Aralkyl-imidazolin-2-one).

Als Beispiele für Verbindungen der allgemeinen Formel 1 seien genannt:
1-Phenyl-4-morpholino-imidazolin-2-on
1-(4-Methoxy)-4-piperidino-imidazolin-2-on
1-(4-Chlorphenyl)-4-morpholino-imidazolin-2-on
1-(4-Chlorphenyl)-4-piperidino-imidazolin-2-on
1-(4-Chlorphenyl)-4-dimethylamino-imidazolin-2-on
1-(4-Bromphenyl)-4-morpholino-imidazolin-2-on
1-(3-Chlorphenyl)-4-morpholino-imidazolin-2-on
1-(4-Chlorphenyl)-4-hexamethylenimino-imidazolin-2-on
1-(4-Chlorphenyl)-4-(4-methylpiperazino)-imidazolin-2-on
1-(4-Methylphenyl)-4-morpholino-imidazolin-2-on
1-(4-Chlorphenyl)-4-(cyclohexyl-methylamino)-imidazolin-2-on
1-(4-Fluorphenyl)-4-morpholino-imidazolin-2-on
1-Benzyl-4-morpholino-imidazolin-2-on

Entsprechend der vorliegenden Erfindung können die Verbindungen der allgemeinen Formel **1** nach einem neuen Verfahren durch Umsetzung der Verbindungen der allgemeinen Formel **2** 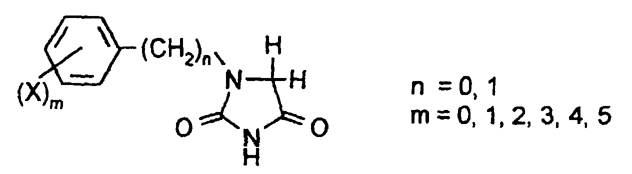 worin
- X =: Wasserstoff, C₁-C₄-Alkyl, C₁-C₄-Alkoxy, Trifluormethyl, Halogen
bedeuten, mit einem sekundären Amin hergestellt werden.

Die Herstellung der Verbindungen der Formel **1** kann wahlweise in einem Lösungsmittel oder in überschüssigen sekundären Amin bei Temperaturen zwischen 50°C und 160°C erfolgen. Als Lösungsmittel kommen bevorzugt aromatische Kohlenwasserstoffe, beispielweise Benzen, Toluen, Chlorbenzen oder Dichlorbenzen in Frage.
Vorzugsweise wird in Gegenwart von wasserbindenden Substanzen wie Zeolithen oder Natriumsulfat gearbeitet. Die Reaktion kann durch Zugabe allgemein üblicher Kondensationskatalysatoren wie 4-Toluensulfonsäure beschleunigt werden.

Die erfindungsgemäßen Verbindungen sind zur Herstellung pharmazeutischer Zusammensetzungen geeignet. Die pharmazeutischen Zusammensetzungen können eine oder mehrere der erfindungsgemäßen Verbindungen enthalten. Zur Herstellung der pharmazeutischen Zubereitungen können die üblichen pharmazeutischen Träger- und Hilfsstoffe verwendet werden.

Die Arzneimittel können parenteral ( zum Beispiel intravenös, intramuskulär subkutan) oder oral angewendet werden.
Die Applikationsformen können nach in der pharmazeutischen Praxis allgemein bekannten und üblichen Verfahren hergestellt werden.

Die erfindungsgemäßen Verbindungen weisen starke antikonvulsive Wirkungen auf.
Sie wurden in vivo nach i.p.-Applikation an Mäusen oder Ratten (p.o.-Applikation) nach dem international üblichen Standard ( Pharmac. Weekblad, Sc.Ed. **14**, 132 (1992) und Antiepileptic Drugs, Third.Ed., Raven Press, New York 1989) auf ihre antikonvulsive Wirkung getestet (Tabelle 1).
Beispielsweise wurde für die Verbindung 2 (1-(4-Chlorphenyl)-4-morpholinoimidazolin-2-on) bei der Ratte für den maximalen Elektroschock die ED₅₀ (p.o.) zu 21 mg/kg, im s.c. Pentetrazol-Test die ED₅₀ zu 16 mg/kg und für die Neurotoxizität die NT₅₀ > 400 mg/kg bestimmt. Im Vergleich dazu wirken bekannte Antiepileptika entweder im Modell maximalen Elektroschock oder im Pentetrazol-Test oder sind bei stärkerer Wirkung im PTZ-Test stark neurotoxisch.

**Tabelle 1**

| Verbindung entsprechend der Beispiele | log P²⁾ | Test³⁾ | Dosis⁴⁾ | Wirkung⁵⁾ |
|---|---|---|---|---|
| 1 | 0,64 | MES | 100 | 30 |
| | | PTZ | 100 | 30 |
| 2 | 1,48 | MES | 300 | 30 |
| | | PTZ | 30 | 70 |
| 3 | 2,29 | MES | 100 | 100 |
| | | PTZ | 100 | 100 |
| 4 | 0,48 | MES | 300 | 30 |
| | | PTZ | 300 | 30 |
| 5 | 2,17 | MES | 300 | 100 |
| | | PTZ | 300 | 100 |
| 6 | 1,61 | MES | 300 | 100 |
| | | PTZ | 100 | 20 |
| 7 | 1,53 | MES | 300 | 100 |
| | | PTZ | 100 | 100 |
| 8 | 1,45 | MES | 300 | 30 |
| | | PTZ | 100 | 100 |
| 9 | 0,97 | MES | 100 | 30 |
| | | PTZ | 100 | 100 |
| 10 | 1,28 | MES | 300 | 10 |
| | | PTZ | 300 | 70 |
| 11 | 2,56 | MES | 300 | 100 |
| | | PTZ | 300 | 40 |

| Vergleichssubstanz | | Test³⁾ | Dosis⁴⁾ | Wirkung⁵⁾ |
|---|---|---|---|---|
| Carbamazepin | | MES | 100 | 100 |
| | | PTZ | 100 | 0 |
| Valproat | | MES | 100 | 0 |
| | | PTZ | 100 | 30 |

| | | | | |
|---|---|---|---|---|
| Anmerkungen zur Tabelle 1: 1) Numerierung der Verbindungen s. Ausführungsbeispiele 2) Verteilungskoeffizient Octanol/Wasser | | | | |
| 3) Maus i.p.: MES = maximaler Elektroschock, PTZ = s.c. Pentetrazol | | | | |
| 4) in mg/kg | | | | |
| 5) in % der geschützten Tiere | | | | |

Die Herstellung der neuen Verbindungen der allgemeinen Formel **1** soll anhand der Ausführungsbeispiele näher erläutert werden.

### Ausführungsbeispiele

Allgemeine Vorschriften zur Herstellung der Verbindungen der Formel **1** gemäß Tabelle 1, Beispiele 1-11

### Variante A

0,05 mol 1-Aryl-imidazolin-2,4-dion der allgemeinen Formel **2** (n=0) 200 mg 4-Toluensulfonsäure zu 100 ml eines entsprechenden sekundären Amins gegeben. Anschließend wird am Soxhlet-Extraktor unter Rückfluß erhitzt, wobei die Extraktionshülse vorher mit ca. 25 g eines wasserbindenden Feststoffes gefüllt wird (geeignet sind calc. Natriumsulfat, Magnesiumsulfat, NaOH, KOH, Zeolithe).
Nach 8 bis 30 Stunden Reaktionszeit wird die Lösung heiß filtriert und etwa bis zum halben Volumen am Rotationsverdampfer destilliert. Die klare Lösung wird im Eisbad gekühlt und der anfallende Kristallbrei vom Amin abgetrennt. Im Rohprodukt enthaltener Ausgangsstoff wird mit 50 ml heißem Aceton extrahiert. Das Produkt wird aus n-Propanol umkristallisiert.
Aus dem abgetrennten Amin kann ca. 0,02 mol unumgesetztes 1-Aryl-imidazolin-2,4-dion zurückgewonnen werden.

### Variante B

0,05 mol 1-Aralkyl-imidazolin-2,4-dion der allgemeinen Formel **2** (n=1) werden mit einem sek. Amin wie unter A beschrieben umgesetzt. Nach 8 bis 30 Stunden Reaktionszeit wird die Lösung heiß filtriert und anschließend am Rotationsverdampfer zur Trockene eingeengt. Zum Rückstand werden 50 ml Methylenchlorid und 50 ml 2n HCl gegeben. Die organische Phase wird abgetrennt und die wässrige Phase noch zwei mal mit Methylenchlorid extrahiert. Die isolierte wässrige Phase wird mit 50 ml 10%iger NaOH alkalisiert und das 1-4-Amino-1-aralkyl-imidazolin-2-on mit 100 ml Methylenchlorid extrahiert. Die Etherextrakte werden über Natriumsulfat getrocknet. Nach dem Abdestillieren des Methylenchlorids wird das Rohprodukt aus Ethanol oder Aceton umkristallisiert.

### Variante C

0,05 mol 1-Ar(alk)yl-imidazolin-2,4-dion der allgemeinen Formel **2** werden mit 100 ml Dimethylammonium-dimethylcarbamat wie unter A und B beschrieben umgesetzt. Nach 40 Stunden Reaktionszeit wird nach Variante A oder B aufgearbeitet.

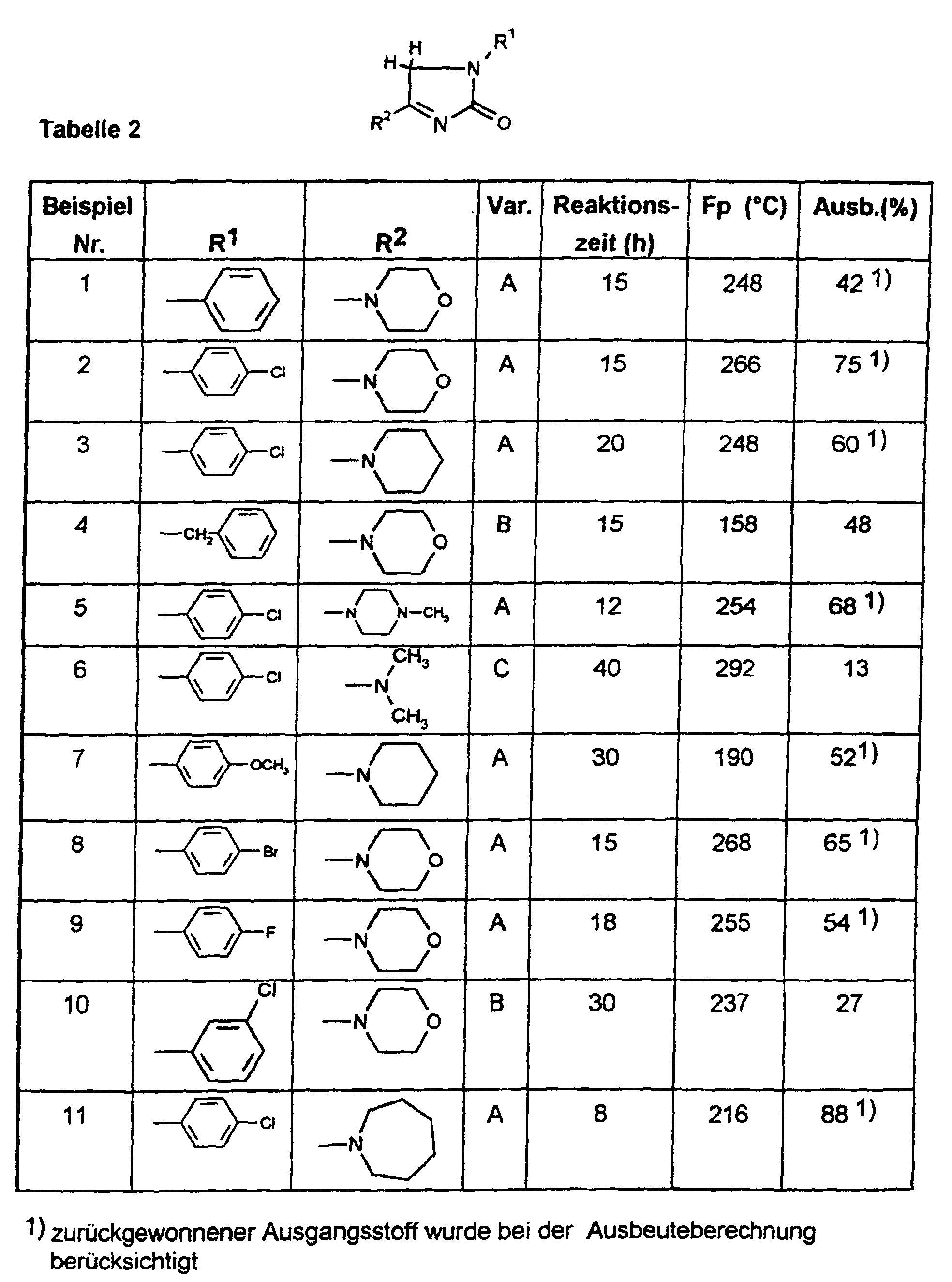

## Patentansprüche

1. Antikonvulsive Verbindungen der allgemeinen Formel 1 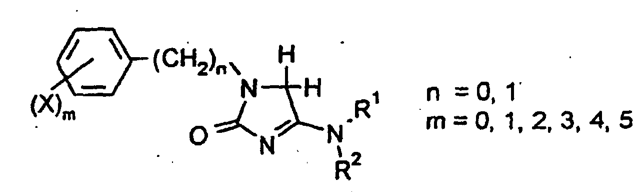 worin
X = Wasserstoff, C₁-C₄-Alkyl, C₁-C₄-Alkoxy, Trifluormethyl, Halogen,
R¹ bzw. R² = C₁-C₄-Alkyl, Cycloalkyl, eine Alkylgruppe, welche ein oder mehrere Heteroatome einschließt,
oder
R¹ und R² zusammen eine Alkylengruppe mit 2 - 6 Kohlenstoffatomen, in dem eine -CH₂-Gruppe durch Sauerstoff, Stickstoff oder Schwefel ersetzt sein kann, oder eine 4-Methylpiperazinogruppe bedeuten.

2. Verbindungen nach Anspruch 1,
1-Phenyl-4-morpholino-imidazolin-2-on
1-(4-Methoxy)-4-piperidino-imidazolin-2-on
1-(4-Chlorphenyl)-4-morpholino-imidazolin-2-on
1-(4-Chlorphenyl)-4-piperidino-imidazolin-2-on
1-(4-Chlorphenyl)-4-dimethylamino-imidazolin-2-on
1-(4-Bromphenyl)-4-morpholino-imidazolin-2-on
1-(3-Chlorphenyl)-4-morpholino-imidazolin-2-on
1-(4-Chlorphenyl)-4-hexamethylenimino-imidazolin-2-on
1-(4-Chlorphenyl)-4-(4-methylpiperazino)-imidazolin-2-on
1-(4-Methylphenyl)-4-morpholino-imidazolin-2-on
1-(4-Chlorphenyl)-4-(cyclohexyl-methylamino)-imidazolin-2-on
1-(4-fluorphenyl)-4-morpholino-imidazolin-2-on
1-Benzyl-4-morpholino-imidazolin-2-on

3. Verfahren zur Herstellung der Verbindungen der allgemeinen Formel **1 dadurch gekennzeichnet, daß** man eine Verbindungen der allgemeinen Formel **2** 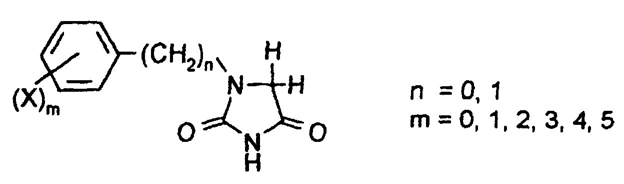 worin
X = Wasserstoff, C₁-C₄-Alkyl, C₁-C₄-Alkoxy, Trifluormethyl, Halogen
bedeutet, mit einem sekundären Amin HNR¹R² bei Temperaturen zwischen 50 ° C und 160 ° C umsetzt, wobei R¹ und R² die angegebene Bedeutung haben.

4. Phamazeutische Zubereitungen **dadurch gekennzeichnet, daß** sie als aktive Substanz mindestens eine Verbindung der allgemeinen Formel **1** und gegebenenfalls pharmazeutische Träger- und Hilfsstoffe enthalten.

5. Phamazeutische Zubereitungen nach Anspruch 4 **dadurch gekennzeichnet, daß** sie als aktive Substanz mindestens eine der Verbindungen gemäß Anspruch 2 enthalten.

6. Verwendung der Verbindungen der allgemeinen Formel **1** zur Herstellung von Arzneimitteln zur Behandlung von epileptischen Erkrankungen.

7. Verwendung der Verbindungen gemäß Anspruch 2 zur Herstellung von Arzneimitteln zur Behandlung von epileptischen Erkrankungen.

## Claims

1. Anticonvulsive compounds of the general formula **1** 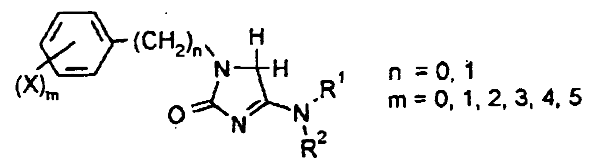 in which
X = hydrogen, C₁-C₄-alkyl, C₁-C₄-alkoxy, trifluoromethyl or halogen,
R¹ and R² = C₁-C₄-alkyl, cycloalkyl or an alkyl group which contains one or more heteroatoms
or
R¹ and R² together are an alkylene group having 2-6 carbon atoms in which a -CH₂- group can be replaced by oxygen, nitrogen or sulphur, or a 4-methylpiperazino group.

2. Compounds according to Claim 1,
1-phenyl-4-morpholinoimidazolin-2-one
1-(4-methoxy)-4-piperidinoimidazolin-2-one
1-(4-chlorophenyl)-4-morpholinoimidazolin-2-one
1-(4-chlorophenyl)-4-piperidinoimidazolin-2-one
1-(4-chlorophenyl)-4-dimethylaminoimidazolin-2-one
1-(4-bromophenyl)-4-morpholinoimidazolin-2-one
1-(3-chlorophenyl)-4-morpholinoimidazolin-2-one
1-(4-chlorophenyl)-4-hexamethyleneiminoimidazolin-2-one
1-(4-chlorophenyl)-4-(4-methylpiperazino)imidazolin-2-one
1-(4-methylphenyl)-4-morpholinoimidazolin-2-one
1-(4-chlorophenyl)-4-(cyclohexylmethylamino)imidazolin-2-one
1-(4-fluorophenyl)-4-morpholinoimidazolin-2-one
1-benzyl-4-morpholinoimidazolin-2-one

3. Process for the preparation of the compounds of the general formula **1, characterized in that** a [sic] compounds of the general formula **2** 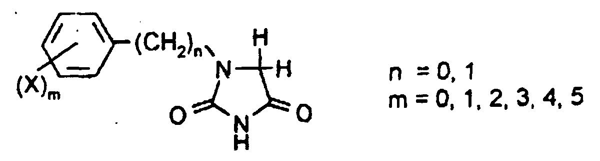 in which
X = hydrogen, C₁-C₄-alkyl, C₁-C₄-alkoxy, trifluoromethyl or halogen
are reacted with a secondary amine HNR¹R² at temperatures between 50°C and 160°C, R¹ and R² having the meaning indicated.

4. Pharmaceutical preparations **characterized in that**, as active substance, they contain at least one compound of the general formula **1** and, if appropriate, pharmaceutical excipients and auxiliaries.

5. Pharmaceutical preparations according to Claim 4 **characterized in that**, as active substance, they contain at least one of the compounds according to Claim 2.

6. Use of the compounds of the general formula **1** for the production of medicaments for the treatment of epileptic disorders.

7. Use of the compounds according to Claim 2 for the production of medicaments for the treatment of epileptic disorders.

## Revendications

1. Composés anti-convulsifs de la formule générale 1 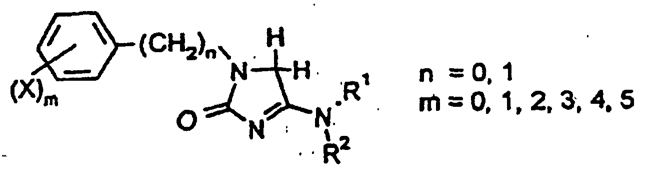 dans laquelle
X = hydrogène, alkyle en C₁ à C₄, alcoxy en C₁ à C₄, trifluorométhyle, halogène,
R¹ ou R² = alkyle en C₁ à C₄, cycloalkyle, un groupe alkyle qui inclut un ou plusieurs hétéroatomes,
ou
R¹ et R² signifient ensembles un groupe alkylène avec de 2 à 6 atomes de carbone, dans lequel un groupe de -CH₂- peut être remplacé par de l'oxygène, par de l'azote ou du soufre, ou un groupe 4-méthylpipérazine.

2. Composés selon la revendication 1,
1-phényle-4-morpholino-imidazolin-2-one
1-(4-méthoxy)-4-pipéridino-imidazolin-2-one
1-(4-chlorophényle)-4-morpholino-imidazolin-2-one
1-(4-chlorophényle)-4-pipéridino-imidazolin-2-one
1-(4-chlorophényle)-4-diméthylamino-imidazolin-2-one
1-(4-bromophényle)-4-morpholino-imidazolin-2-one
1-(3-chlorophényle)-4-morpholino-imidazolin-2-one
1-(4-chlorophényle)-4-hexaméthylèneimino-imidazolin-2-one
1-(4-chlorophényle)-4-(4-méthylepipérazino)-imidazolin-2-one
1-(4-méthylphényle)-4-morpholino-imidazolin-2-one
1-(4-chlorophényle)-4-(cyclohexyle-méthylamino)-imidazolin-2-one
1-(4-fluorophényle)-4-morpholino-imidazolin-2-one
1-benzyle-4-morpholino-imidazolin-2-one

3. Procédé pour la préparation des composés de la formule générale 1, **caractérisé en ce que** l'on fait réagir un composé de la formule générale 2 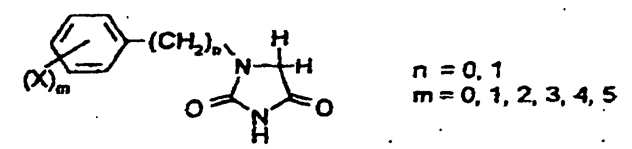 dans laquelle
X signifie hydrogène, alkyle en C₁ à C₄, alcoxy en C₁ à C₄, trifluorométhyle, halogène,
avec un amine secondaire HNR¹R², à des températures situées entre 50° C et 160° C,
R¹ et R² ayant la signification précitée.

4. Préparations pharmaceutiques, **caractérisées en ce qu'**en tant que principe actif, elles contiennent au moins un composé de la formule générale 1 et le cas échéant, des véhicules et auxilaires pharmaceutiques.

5. Préparations pharmaceutiques selon la revendication 4, **caractérisées en ce qu'**en tant de principe actif, elles contiennent au moins l'un des composés selon la revendication 2.

6. Utilisation des composés de la formule générale 1 pour la préparation de médicaments destinés à traiter des maladies épileptiques.

7. Utilisation des composés selon la revendication 2, pour la préparation de médicaments destinés à traiter des maladies épileptiques.
